# EUROPEAN PATENT APPLICATION

(11) **EP 1 741 791 A2**
(43) Date of publication of application: **10.01.2007**
(21) Application number: 04805142.9
(22) Date of filing: 03.12.2004
(51) Int. Cl.: C12Q 1/68

(54) **METHOD AND KIT FOR THE GENOTYPIFICATION OF HLA-B27 BASED ON REAL TIME PCR**

(30) Priority: 17.12.2003 ES 200400009
(71) Applicant: UNIVERSITAT AUTONOMA DE BARCELONA, 08193 Bellaterra (ES)
(72) Inventor: JUAN OTERO, Manuel, LIRAD-SSR-CTBT, 08916 Badalona (Barcelona) (ES); CASAMITJANA PONCES, Natàlia, LIRAD-SSR-CTBT, 08916 Badalona (Barcelona) (ES); PUJOL BORRELL, Ricardo, LIRAD-SSR-CTBT, 08916 Badalona (Barcelona) (ES); COLOBRAN ORIOL, Roger, LIRAD-SSR-CTBT, 08916 Badalona (Barcelona) (ES); PALOU RIVERA, Eduardo, LIRAD-SSR-CTBT, 08916 Badalona (Barcelona) (ES); FANER CANET, Maria Rosa, LIRAD-SSR-CTBT, Badalona (Barcelona) (ES); RIBERA CRUSAFONT, Ana, LIRAD-SSR-CTBT, Badalona (Barcelona) (ES)
(74) Representative: Barlocci, Anna
(86) International application number: PCT/ES2004/070105
(87) International publication number: WO 2005/059505

(57) **Abstract**

The invention relates to a method for determining alleles of the 27 allelic group of B locus of human leukocyte antigen (HLA-B27) from a nucleic acid sample and to a kit for performing this method. The kit comprises allele-specific primers for using in real-time polymerase chain reaction (PCR), and allele-specific fluorescently labelled probes or an intercalating agent, for detecting fluorescence signals. The analysis is completed with the determination of the melting temperatures of the amplified nucleic acid sequences. It overcomes some of the limitations of conventional DNA-based typing methods, in terms of time, possibility of automation, and increase in resolution, while reducing the number of PCR reactions. It is useful e.g. for determining susceptibility for a specific disease.

SEQUENCE LISTING

<110>  Universitat Autonoma de Barcelona

<120>  Method and kit for HLA-B27 genotyping based on real-time PCR

<130>  P560EP00

<150>  P200400009
<151>  2003-12-17

<150>  PCT/ES2004/070105
<151>  2004-12-03

<160>  22

<170>  PatentIn version 3.3

<210>  1
<211>  17
<212>  DNA
<213>  Homo sapiens

<400>  1
tacgtggacg acacgct                                                    17

<210>  2
<211>  15
<212>  DNA
<213>  Homo sapiens

<400>  2
gtgccttggc cttgc                                                      15

<210>  3
<211>  20
<212>  DNA
<213>  Homo sapiens

<400>  3
agtctgtgtg ttggtcttgc                                                 20

<210>  4
<211>  21
<212>  DNA
<213>  Homo sapiens

<400>  4
tacggctgtc atcacttaga c                                               21

<210>  5
<211>  18
<212>  DNA
<213>  Homo sapiens

<400>  5
cttcatccac gttcacct                                                   18

<210>  6
<211>  16
<212>  DNA
<213>  Homo sapiens

<400>  6
gccccgcttc atcacc                                                        16


<210>  7
<211>  20
<212>  DNA
<213>  Homo sapiens

<400>  7
cacgtcgcag ccatacatat                                                    20


<210>  8
<211>  15
<212>  DNA
<213>  Homo sapiens

<400>  8
gcccgcggct cctct                                                         15


<210>  9
<211>  17
<212>  DNA
<213>  Homo sapiens

<400>  9
tccagggtct cacaccc                                                       17


<210>  10
<211>  16
<212>  DNA
<213>  Homo sapiens

<400>  10
cggcggtcca ggagct                                                        16


<210>  11
<211>  16
<212>  DNA
<213>  Homo sapiens

<400>  11
ccgcggtcca ggagct                                                        16


<210>  12
<211>  21
<212>  DNA
<213>  Homo sapiens

<400>  12
agaggacctg ccggaccctg c                                                21

<210>  13
<211>  25
<212>  DNA
<213>  Homo sapiens

<400>  13
gctactacaa ccagagcgag gccgg                                            25

<210>  14
<211>  19
<212>  DNA
<213>  Homo sapiens

<400>  14
ggagcattgg gaccgggag                                                   19

<210>  15
<211>  23
<212>  DNA
<213>  Homo sapiens

<400>  15
cgccgtggat agagcaggag ggg                                              23

<210>  16
<211>  18
<212>  DNA
<213>  Homo sapiens

<400>  16
ggtaccacca gcacgcct                                                    18

<210>  17
<211>  24
<212>  DNA
<213>  Homo sapiens

<400>  17
cgacggcaag gattacatcg ccct                                             24

<210>  18
<211>  20
<212>  DNA
<213>  Homo sapiens

<400>  18
tgaggttcga cagcgacgcc                                                  20

<210>  19
<211>  19
<212>  DNA

<213>  Homo sapiens

<400>  19
cgagtccgag agaggagcc                                                    19


<210>  20
<211>  35
<212>  DNA
<213>  Homo sapiens

<400>  20
caaacagaca ccatggtgca cctgactcct gagga                                  35


<210>  21
<211>  29
<212>  DNA
<213>  Homo sapiens

<400>  21
aagtctgccg ttactgccct gtggggcaa                                         29


<210>  22
<211>  16
<212>  DNA
<213>  Homo sapiens

<400>  22
tgtgccttgg ccttgc                                                       16

## Description

This invention relates to the field of medicine generally and specifically to medical research, molecular biology, forensics and diagnostics. In particular, the invention provides a method for determining the genotype (i.e. the specific set of alleles) of an individual. This facilitates determining e.g. the susceptibility for a specific disease of an individual.

### BACKGROUND ART

The genes that encode the human leukocyte antigens (HLA) system are among the most variable genes in humans: each variant codes for molecules which bind to different set of peptides. These genes are the same in all the cells of a particular individual, but differ from person to person. HLA comprises several genes clustered on a 4 Mb-segment of the short arm of chromosome 6 (cf. D.A. Rhodes et al., "Genetics and molecular genetics of the MHC", Rev. Immunogenet. 1999, vol. 1, pp. 21-31). The HLA region comprises six major loci that encode structurally homologous proteins which are classified into HLA class I (HLA-A, B, Cw) and class II (HLA-DR, DQ, DP), that present antigens to two different subsets of cells. HLA Class I molecules present antigens to T cells that express the CD8 cell surface glycoprotein.

In addition to functioning as strong transplantation antigens, HLA-A, B and C also play a crucial role in the presentation of viral antigens, tumor antigens, and haptens to cytotoxic T lymphocytes. Also, paradoxically, the inheritance of certain class I haplotypes appear to predispose individuals to chronic familial diseases of unknown etiology. The prevalence of the HLA-B27 alleles in Caucasian population ranges from 3 to 8%. HLA-B27 itself is a serologic specificity that encompasses 24 different alleles. The HLA-B27 alleles seem to have evolved from the most widespread subtype, B2705. The great clinical interest of these alleles relies on its strong association to a group of rheumatic diseases, the spondylarthropathies, with Ankylosing Spondylitis (AS) as a disease entailing a 95% of relative risk. There is currently no satisfactory explanation for the association of HLA-B27 with AS, but HLA-B27 specificity has become the best marker for the diagnosis of these group of diseases for which there are no reliable autoantibodies or biological markers. Case-control studies established that subtypes 2705, 2702, 2707, and 2704 have a clear and strong association with disease, although B2705 is the only one with widely distributed in the population. Subtypes B2709 and B2706 are not associated to AS in certain populations (Sardinian and Thais respectively), and B2703 association with AS remains unclear among West Africans.

HLA genotyping techniques based on polymerase chain reaction (PCR) have become a widely used alternative to serological methods in clinical practice. The most common PCR-based HLA typing methods that overcome serological tests limitations are PCR with sequence-specific primers (PCR-SSP) and PCR with sequence-specific oligonucleotide hybridization (PCR-SSO). In PCR-SSP methodology, gene sequence is determined by amplifying the hypervariable region of target HLA antigen to determine the HLA antigen type. In PCR-SSO method, a membrane (on which DNA amplified by primers specific to HLA gene is immobilized), is prepared and respective HLA type specific oligonucleotide probes are hybridized for typing (cf. EP 514.534; EP 417.160 and R.K. Saiki et al., "Analysis of enzymatically amplified beta-globin and HLA-DQ alpha DNA with allele-specific oligonucleotide probes", Nature 1986, vol 324, pp. 163-6). Usually, HLA-B27 typing is carried out by PCR-SSP (cf. O. Olerup, "HLA-B27 typing by a group-specific PCR amplification", Tissue Antigens 1994, vol. 43, pp. 253-6), and subtyping requires several additional PCR-SSP (cf. S. García-Fernández et al., "New insights regarding HLA-B27 diversity in the Asian population", Tissue Antigens 2001, vol. 58, pp. 259-62), PCR-SSO or amplified fragment length polymorphism (AFLP) procedures. PCR-sequence-based typing (PCR-SBT) is also used to resolve ambiguities (cf. EP 910.662). Thus, even if an approximate definition of the HLA-B27 genotype of a sample can be obtained using a one step assay, the resolution of the 24 B27 alleles requires additional time-consuming tests.

While all these methods have been effective in the routine laboratory practice they require time-consuming post-PCR processing; besides post-PCR contamination is a real risk. Moreover, PCR-SSP requires the use of a large number of PCR reactions and it is difficult to automate, thus limiting its throughput. On the other hand, even if PCR-SSO is capable of a higher throughput, its processing times are longer and can not discriminate among motifs occurring on cis-trans strands of the DNA template. Due to the fact that HLA genotyping laboratories need to have feasible clinical results for the detection of rheumatic diseases such as ankylosing spondylitis (AS), it is highly desirable to provide new typing methods, particularly if they are improved in the sense of they are faster, are potentially automated, give high resolution and reduce contamination risks.

### SUMMARY OF THE INVENTION

Some previous studies have reported that real-time PCR combined with FRET (see definition below) probes provides a potentially powerful tool to overcome some of the above mentioned limitations (cf. EP 1.229.128 A1). Also, some protocols for typing HLA-B27 by real-time PCR with SYBR^{®} green I have been developed to obviate the gel electrophoresis step and to shorten processing time (cf. C. Tiemann et al., "Rapid DNA typing of HLA-B27 allele by real-time PCR using lightcycler technology", Clin. Lab. 2001, vol 47, pp. 131-4). But these protocols do not allow subtyping or allele-specifically analysing to avoid ambiguous results. In the present invention, inventors have taken advantage of the potential of FRET real-time PCR for HLA-B27 variant typing.

Thus, an aspect of the present invention relates to a method for determining alleles of the allelic group HLA-B27 from a nucleic acid sample, comprising the steps of: (i) amplifying any nucleic acid that comprise a part of the region exon 2-intron 2-exon 3 of HLA-B locus by real-time polymerase chain reaction (PCR); and (ii) detecting fluorescence signals either by fluorescently labelled nucleotide probes or by an intercalating agent, after the amplification carried out in step (i), and analysing melting temperatures (i.e. negative fluorescence derivative divided by temperature, -d(F1)/dT) of the amplified nucleic acid sequences. When detection in step (ii) has been done by fluorescently labelled nucleotide probes, the method continues with a further step (iii) comparing more than one signal detected in step (ii) with an experimentally defined fluorescence pattern; said pattern having been established by an initial definition based on theoretical comparison of the sequences of probes of step (ii) with the sequences of the different alleles of the allelic group, followed by a definitive definition based on an experimental assessment of which of the signals are actually positive for each melting temperature (black squares in FIG. 5), which are actually negative for each melting temperature (white squares in FIG. 5), or which are actually negative for lack of amplification (grey squares in FIG. 5). In all cases, steps (i) and (ii) of this method are carried out in the same tube and without any further manipulation of the sample.

In general, the nucleic acid in the sample will be DNA, most usually genomic DNA. However, the method of the present invention can also be practiced with other nucleic acids, such as messenger RNA or cloned DNA, and the nucleic acid may be either single-stranded or double-stranded.

Another aspect of the present invention refers to a kit for determining alleles of the HLA-B27 allelic group from a nucleic acid sample by performing the method described above. The kit comprises: (i) primers for amplifying nucleic acids that comprise a part of the region exon 2-intron 2-exon 3 of HLA-B locus by real-time PCR; and (ii) either fluorescently labelled nucleotide probes or an intercalating agent, for detecting fluorescence signals after the amplification; and, when fluorescently labelled nucleotide probes are included, further comprising (iii) experimentally defined fluorescence patterns for comparing the detected signals as it is described above.

Real-time PCR monitors the fluorescence emitted during the reaction as an indicator of amplimer production during each PCR cycle (i.e., in real time), as opposed to the endpoint detection by conventional quantitative PCR methods. The real-time PCR does not detect the size of the amplimer and thus does not allow the differentiation between DNA and cDNA amplification. The real-time PCR system is based on the detection and quantification of a fluorescent reporter.

In a particular embodiment of the invention, the kit provides primers which are single strand nucleic acids, which have a length from 10 to 30 nucleotides, particularly from 14 to 24, and which comprise sequences complementary to sequences of a region located along exon 2, intron 2 and exon 3 of HLA-B locus. In a preferred embodiment, primer sequences are selected from the group consisting of SEQ ID NO: 1-3, SEQ ID NO: 6-11 and SEQ ID NO: 22. The amplifying step (i) of the method is carried out with two or more primers. Real-time PCR is performed using universal cycling thermal parameters and PCR reaction conditions known for a skilled in the art.

The kit of the invention also provides probes which are single strand nucleic acids, which have a length from 10 to 35 nucleotides, particularly from 15 to 27 nucleotides, and which comprise sequences complementary to sequences of a region located along exon 2, intron 2 and exon 3 of HLA-B locus. In a preferred embodiment, probe sequences are selected from the group consisting of SEQ ID NO: 12-21 or the complementary sequences of this group. In a particular embodiment, probes are labelled with fluorochromes of type donor label-acceptor label which induces the phenomenon called FRET (Förster or fluorescence resonance energy transfer). When irradiated, the excited fluorescent label (donor) transfers energy to the nearby acceptor label molecule rather than fluorescing. The fluorescence resonance energy transfer is the magnitude which allows to monitor the amplimer production in the PCR real-time. Specifically labels for this purpose are fluorescein, LC-RED 640™ and LC-RED 705™ from Tib-Mol Biol. Assays can be performed using multiple labels with distinct emission wavelengths.

Alternatively to the use of sequence specific fluorescent probes, in another particular embodiment, an intercalating agent is used for detecting fluorescence signals of the amplification. In a preferred embodiment, the intercalating agent is SYBR green I from Molecular Probes. SYBR green I is a minor groove binding dye. In the case of using an intercalating agent, the binding with the PCR product is non-specific.

In another particular embodiment, the kit furhter comprises two primers for β-globin gene with SEQ ID NO: 4 and SEQ ID NO: 5, added as a control. This control can be external (in a different tube), but it is preferably internal (in the same tube). The kit can optionally comprise instructions for determining the alleles of HLA-B27 of a sample according to kit ingredients.

Based on the method of the present invention it is possible to design a protocol to determine the majority of alleles belonging to HLA-B27 allelic group. Just in two real-time PCR reactions, the frequent B27 alleles in the population can be determined and an even higher clinically relevant discrimination level can be obtained by the inclusion of a third PCR reaction. All these reactions could be done in the same time in three parallel tubes. In the first PCR reaction, which can be called "typing", a group specific amplification goes from the beginning to the middle of exon 2, the common region of B27 alleles. This first reaction allows to confirm the presence of HLA-B27 in the sample. The second amplification, which can be called "subtyping", goes from the beginning of exon 2 to the beginning of exon 3 (including intron region and all the highly polymorphic region in the end of exon 2). And the third amplification, which can be called "refining", analyzes the highly polymorphic region in the middle of exon 3. This last reaction allows to determine infrequent allelic variants of HLA-B27. Primers and fluorescently labelled nucleotide probes of the kit of the present invention allows to carry out the three mentioned reactions: typing, subtyping and refining, while an intercalating agent, due to its unspecificity, allows to carry out only the first reaction where the presence of HLA-B27 is confirmed.

The method and kit of the present invention allow to identify the main HLA-B27 alleles by allocating them in eight or twelve groups, thus bringing the test to an almost-high typing resolution. Taking into account HLA-B27 allele frequencies in the population, this test makes possible to separate those alleles associated to Ankylosing Spondylitis (2705, 2702, 2704, 2707) from those not clearly associated (2709, 2706 and 2703). The genotyping method of the present invention and the kit for performing the method have some advantages respect to methods and kits known in the art. The method is shorter than other PCR approaches previously described (90 minutes including analysis), reaching also a higher resolution than other conventional HLA-B27 PCR-SSP genotyping assays. Few reactions are required to complete the analysis, two reaction tubes being sufficient for a good level of typing resolution, and three reaction tubes for a high degree of allelic definition. No post-PCR steps are required thus avoiding handling of the PCR amplimer. Thus, the method of the present invention have the required reproducibility, precision and simplicity for diagnostic purposes. Inter-assay variation of Tm is always below 1 °C, and below 0.3 °C at intra-assay replicates; and all PCR-reactions (B27-detection, B27-subtyping and B27-refining) can be run in 55 cycles in the same reaction and in the same conditions.

Terms "genotyping" and "typing" are used as synonymous in this description and they refer to any testing that reveals the specific alleles inherited by an individual, particularly useful for situations in which more than one genotypic combination can produce the same clinical presentation. In the present description, the term "locus" means the position occupied by each HLA gene (e.g. B locus of HLA). The term "allele" refers to one of two or more alternative forms of a gene, differing in genetic sequence and resulting in observable differences in heritable character (phenotype), and are found at the same place on a chromosome. In the case of HLA-B locus, HLA-B27 is an allelic group which is constituted by several alleles, e.g. HLA-B2705 or HLA-B2709.

The genotyping method of the present invention facilitates e.g. determination of the susceptibility of an individual for a specific disease. As mentioned above, compared with some HLA typing methods known in the art, the method of the present invention is faster and easier to automate, it gives higher resolution and it reduces sample contamination risks.

Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration, and are not intended to be limiting of the present invention.

### BRIEF DESCRIPTION OF THE DRAWINGS

FIG. 1 (1A-1F) illustrates HLA-B27 detection reaction and the melting profiles observed.
FIG. 2 (2A-2C) illustrates HLA-B27 subtyping amplification, melting profiles observed, sequence of S1 probes set, and mismatched alleles.
FIG. 3 (3A-3C) illustrates HLA-B27 subtyping amplification, melting profiles observed, sequence of S2 probes set, and mismatched alleles.
FIG. 4 (4A-4C) illustrates HLA-B27 refine amplification, melting profiles observed, sequence of S3 probes set, and mismatched alleles.
FIG. 5 is a table of the specificities of reactions and probes used in the three reactions of HLA-B27 genotyping. Black squares indicate specificity and grey squares indicate no amplification. For B27 detection specificities Tm is 91 °C when monitored with SYBR green I, and 68 °C when monitored with detection probes.

### DETAILED DESCRIPTION OF PARTICULAR EMBODIMENTS

### Detailed description of Figures

FIG. 1A depicts detection reaction during the real-time PCR by representing fluorescence (F1) vs. cycle number (N). F1 indicates fluorescence monitored at 510-550 nm. FIG. 1 B depicts the melting profiles observed by representing the negative fluorescence derivative divided by temperature (-d(F1)/dT) vs. temperature (T, in °C). The melting temperature for beta-globin is 89 °C, and for HLA-B27 is 91 °C. FIG. 1C depicts detection reaction during the real-time PCR by representing fluorescence (F2/F1) vs. cycle number (N). F2 indicates fluorescence monitored at 620-660 nm. FIG. 1D depicts the melting profiles observed by representing -d(F2/F1)/dT vs. temperature (T, in °C). FIG. 1 E depicts detection reaction during the real-time PCR by representing fluorescence (F3/F1) vs. cycle number (N). F3 indicates fluorescence monitored at 670-750 nm. FIG. 1 F depicts the melting profiles observed by representing -d(F3/F1)/dT vs. temperature (T, in °C). FIG. 1A and 1 B are from detection reaction using SYBR green I, and FIG. 1C-F using detection probes.

FIG. 2A depicts detection reaction during the real-time PCR by representing fluorescence (F2/F1) vs. cycle number (N). FIG. 2B depicts the melting profiles observed by representing -d(F2/F1)/dT vs. temperature (T, in °C). FIG. 2C depicts sequence of S1 probes set and the mismatched alleles. Samples with 0 mismatch are shaded in grey; samples with 2 mismatches are indicated with a continous line box; samples with 3 mismatches are no shaded; and samples with 4 mismatches are indicated with a pointed line box.

FIG. 3A depicts detection reaction during the real-time PCR by representing fluorescence (F3/F1) vs. cycle number (N). FIG. 3B depicts the melting profiles observed by representing -d(F3)/dT vs. temperature (T, in °C). FIG. 3C depicts sequence of S2 probes set and the mismatched alleles. Samples with 0 mismatch are no shaded; samples with 1 mismatch are shaded in grey; samples with 2 mismatches are indicated with a pointed line box; and samples with 3 mismatches are indicated with a single line box.

FIG. 4A depicts detection reaction during the real-time PCR by representing fluorescence (F2/F1) vs. cycle number (N). FIG. 4B depicts the melting profiles observed by representing -d(F2)/dT vs. temperature (T, in °C). FIG. 4C depicts sequence of S3 probes set and the mismatched alleles. Samples with 0 mismatch are indicated with a single line box; samples with 1 mismatch are shaded in grey; and samples with 3 mismatches are no shaded.

### Sample preparation

For the setting of the typing method, nine HLA-B27+ lymphoblastoid EBV transformed cell lines homozygous and heterozygous (TABLE 1) for the HLA-B-2701 to 2709 alleles (LH, R34, CH, WE-1, HOM-2, LG-2, PAR, RQ, Cl; provided by Dr. Lopez de Castro, Madrid) were used as positive controls. Cells were grown in tissue culture medium RPMI 1640 (Whittaker, Waskerville, MD) containing 10% foetal calf serum (FCS, Gibco) 2 mM L-glutamine (Life Technologies, Merelbeke, Belgium) and 100 UI/ml penicillin (Normon laboratories, Spain) and 0.1 mg/ml streptomycin (CEPA, Spain).

For the validation of the method, a total of 60 clinical samples from bone marrow, solid organ transplantations donors or from healthy individuals, half of them known to be HLA-B27 positive, were analyzed by the new technique.

Genomic DNA was obtained by a standard salting-out procedure (cf. S.A. Miller et al., "A simple salting out procedure for extracting DNA from human nucleated cells", Nucleic Acids Res. 1988, vol 16, pp. 1215), and diluted (up to 0.05 µg/ml) with TE buffer (10 mM Tris-HCl, pH 8.0, 1 mM EDTA, pH 8.0). All samples (cell lines and donor cells) were typed previously for HLA-B27 by a PCR-SSP and reanalyzed by PCR-SBT.

### Real-time PCR reaction for the set up and the validation of the method

All the real-time PCR reactions were performed in a LightCycler™ Instrument (Roche) by combining amplification with specific primers (sequences specified in TABLE 2) with fluorochrom detection.

### A) B27 detection, carried out by two different ways, using detection probes or

SYBR green I: The first one consists in a PCR reaction with specific primers and monitored by FRET probes (B27 detection anchor probe was labeled with LC-Red 640™ and B27 detection sensor probe was labeled with fluorescein). As DNA amplification control, β-globin gene specific primers were used (β-globin S and β-globin AS), generating a 248 bp product which production was also monitored with FRET probes. CTW FL anchor probe was labeled with LC-Red 705™ and CTW FL sensor probe was labeled with fluorescein (cfr. C.T. Wittwer et al., "Continuous fluorescence monitoring of rapid cycle DNA amplification", Biotechniques 1997, vol. 22, p. 130).

For the B27 detection, specific primers were used to obtain a 140 bp HLA-B27 specific amplification, although it excluded the unusual 2718 and 2723 alleles. As a sense primer, 149c was used and as an antisense a mix of 273c and 273c3 (the numbers 149, 273 indicate positions 3' respect the cDNA). The PCR reaction was performed in a total volume of 10 µl; the reaction mixture contained 0.2 µM of 149C primer, 0.6 µM of 273C primer, 0.2 µM of 273C3 primer, 0.2 µM of β-globin S primer, 1 µM of β-globin AS primer, 0.2 µM of each hybridization probe, 1 µl of Light Cycler Fast Start Master Hybridization probes^{™}, 2 µl of DNA template and a final concentration of 4 mM MgCl₂. The real time amplification protocol for this second reaction was: 10 min of initial denaturation and activation of the Fast Start^{™} enzyme at 95 °C, followed by 55 amplification cycles (15 s of denaturation at 95 °C, 5 s of annealing at 67 °C and 20 s of extension at 72 °C). Melting curves were generated at 95 °C 0 s, 72 °C 20 s, 65 °C 120 s, 35 °C 0 s (all at ramp rate 20 °C/s) and 80 °C 0 s (ramp rate 0.1 °C/s; acquisition mode: continuous), followed by a cooling step of 30 s at 40 °C.

As alternative to the use of FRET probes for monitoring the B27 and β-globin amplification the reaction has been optimized with the use of SYBR^{®} Green I dye, this version of B27-detection has been designed as (B27-SYBR-detection). We have also developed an alternative approach monitored with SYBR green I, for the detection of all HLA-B27 alleles, although this reaction includes the HLA-B73 allele; this alternative approach was designed as B27-Alternative (ap).In both SYBR green I approaches, PCR reaction was performed in a total volume of 10 µl and the reaction mixture contained: 0.15 µM of 149c and 273c-mix (0.2 µM of p127c and 0.2 µM of 204c in the ap), 0.3 µM of each β-globin specific 0.15 µM of 149c and 273c-mix (0.2 µM of p127c and 0.2 µM of 204c in the ap), 0.3 µM of each β-globin specific primer, 1µl of LightCycler Fast Start SYBR^{®} green 1, 2 µl of sample DNA (usually up to 100 ng), and a final concentration of 2 mM MgCl₂ (4 mM MgCl₂ in the ap).

The real-time-PCR amplification parameters for this reaction were: 10 min of initial denaturation and activation of the Hot-star Taq (Fast Start^{™} Roche) enzyme at 95 °C, followed by 55 amplification cycles (15 s of denaturation at 95 °C, 5 s of annealing at 67 °C and 20 s of extension at 72 °C). Melting curves for (B27-SYBR-detection) were generated at 95 °C 0 s, 75 °C 15 s ramp rate 20 °C/s, and 99 °C ramp rate 0.1 °C/s (acquisition mode: continuous). For the ap, cycle conditions showed some little but important changes: 45 amplification cycles, 10 s of denaturation at 95 °C, 5 s of annealing at 69 °C and 15 s of extension at 72 °C. Melting curve was generated at 95 °C 0 s, 67 °C 15 s ramp rate 20 °C/s, and 99 °C ramp rate 0.05 °C/s (acquisition mode: continuous).

"B27 detection reaction" in both variants (with probes or with SYBR green I) distinguished HLA-B27 positive from negative samples, combining real-time PCR amplification and analysis of melting curves. In the case of SYBR detection, the negative samples gave a single Tm, corresponding to the beta-globin (at 88 °C) while HLA-B27-positive samples gave two peaks (at 88 and 91 °C, FIG. 1B). In the detection with B27 probes and β-globin probes, B27 detection through the F2 fluorescence channel, amplification of HLA-B27+ alleles (except 2718 and 2723) were detected (FIG. 1C). Melting curves generated a single pick at 67.5 °C only for HLA-B27+ samples (FIG. 1 D). Positive samples could reliably be distinguished from HLA-B27 negative alleles during both steps of reaction, with F2 monitoritzation. By monitoring the CTW probes through the F3 channel, amplification of β-globin was detected in all samples (FIG. 1E). Melting curves generated one or two melting profiles (71 °C and 75 °C) corresponding to the presence of two variants of β-globin gene (FIG. 1F). These variants did not interfere in the analysis of HLA-B27 alleles.

When 100% positive allele identification is required (not the case for AS diagnosis) the alternative approach described could be used. Inter-assay variation for the reaction described below had always been below 1 °C (a maximum of 3 ° C is usually accepted).

B) B27 subtyping: The second set of reactions used FRET probes to define allelic variants of B27. As a main FRET PCR amplification (B27 subtyping) with 127C and 362C primers, a 494 bp fragment was obtained in all HLA-B27 alleles, except for 2707, 2711, 2714, 2719, 2721 and 2724 (six low frequency alleles) variants. For allelic discrimination, two sets of FRET hybridization probes were used: the most discriminating set was designated as S1, and the other pair of probes as S2. For S1 probes, anchor probe was labeled with LC-Red 640™ and sensor probe was labeled with fluorescein. For S2 probes, anchor probe was labeled with fluorescein and sensor probe was labeled with LC-Red 705™. The PCR reaction was performed in a total volume of 10 µl, the reaction mixture contained 0.2 µM of 127C primer, 1 µM of 362C primer, 0.2 µM of each hybridization probe, 1 µl of Light Cycler Fast Start Master Hybridization probes^{™}, 2% DMSO PCR grade (SIGMA), 2 µl of template DNA and a final concentration of 3.5 mM MgCl₂. The real-time amplification protocol for this second reaction was: 10 min of initial denaturation and activation of the Fast Start^{™} enzyme at 95 °C, followed by 55 amplification cycles (15 s of denaturation at 95 °C, 5 s of annealing at 67 °C and 20 s of extension at 72 °C). Melting curves were generated at 95 °C 0 s, 72 °C 20 s, 65 °C 120 s, 35 °C 0 s (all at ramp rate 20 °C/s) and 80 °C Os (ramp rate 0.1 °C/s; acquisition mode: continuous), followed by a cooling step of 30 s at 40 °C.

"B27 subtyping" reaction allowed allelic discrimination of eight B27 allelic groups, a level of definition acceptable for most purposes. This reaction detected the majority of HLA-B27 alleles with the exception of some low frequency ones (FIG. 5). By acquiring S1 melting through the F2 fluorescence channel, four Tm could be defined: 69 °C, 62 °C, 50 °C and 44 °C (FIG. 2A). The reduction of the Tm was proportional to the number of mismatches (0, 2, 3, 4) of the amplimer with the S1 sensor probe as shown in FIG. 2B. By monitoring the S2 probes melting curves through the F3 channel, two melting profiles were defined: most B27 alleles were detected as a peak at 60 °C, while B2703 was detected by the peak at 69 °C, as is shown in FIG. 3A.

C) B27 refine: By "B27 subtyping" reaction, HLA-B27 alleles could be separated into eight groups. To gain a higher level of resolution and distinguish non-AS associated alleles, a third real-time PCR protocol was designed. An additional reaction was performed using 344C and a mix of 462d and 462 primers amplifying a 140 bp fragment in all HLA-B27 except for 2707, 2711, 2714, 2719, 2720 and 2724 variants (low frequency alleles) and other non HLA-B27 alleles (FIG. 5).

Due to the fact that some other amplified alleles by PCR do not give fluorescent signal because of their high number of mismatches with the sensor probe, they have been considered as non amplified alleles. For the allelic discrimination, a set of FRET hybridization probes was used, S3: anchor probe was labeled with fluorescein and sensor probe labeled with LC-Red 640™. The PCR reaction was performed in a total volume of 10 µl, the reaction mixture contained 0.2 µM of 344C primer, 0.6 µM of a mix of 462 antisense primers, 0.2 µM of each hybridization probe, 1 µl of Light Cycler Fast Start Master Hybridization^{™} probes, 2 µl of template DNA and a final concentration of 3 mM MgCl₂. The real-time amplification protocol for this reaction was the same as for the B27-subtyping reaction: 10 min of initial denaturation and activation of the Fast Start^{™} enzyme at 95 °C, followed by 55 amplification cycles (15 s of denaturation at 95 °C, 5 s of annealing at 67 °C and 20 s of extension at 72 °C). Melting curves were generated at 95 °C 0 s, 72 °C 20 s, 65 °C 120 s, 35 °C 0 s (all at ramp rate 20 °C/s) and 80 °C 0 s (ramp rate 0.1 °C/s; acquisition mode: continuous), followed by a cooling step of 30 s at 40 °C.

The addition of a "B27 refine" reaction allowed allelic discrimination of twelve B27 alleles. By acquiring S3 melting curves through F2 channel fluorescence, three Tm were defined at 66 °C, 55 °C and 38 °C (FIG. 4A). The decrease of Tm in each case was proportional to the number of mismatches (0, 1, 3), of the target sequence with the S3 sensor probe shown in FIG. 4B. A 38 °C Tm was defined for B2706 and B2721, 66 °C for B2709 and 55 °C for the other B27 subtypes (except B2707, B2711, B2714, B2719, B2720 and B2724 non-amplified alleles). Combining these results with the previous two-reaction approach gave twelve B27 allelic groups (FIG. 5).

### Heterozygosis indeterminations analysis

Although it is really unusual to find B27 alleles in the two haplotypes of the same patient, a computer simulation using Hetero software (cf. M. Juan et al., "Análisis informático de las técnicas de genotipificación de los antígenos HLA de clase II: HETERO v2.0", Inmunología 1992, vol. 11, pp. 14-20) was carried out to analyze whether the coexistence of different HLA-B27 allelic variants in heterozygosis would produce additional typing indetermination. Briefly, this software allow to combine the typing patterns (coded in binary form where 1 codes for detection and 0 for non-detection) of each allele, to indicate if a given heterozygous combination has additional undistinguishable patterns.

### Comparison with other typing methods

To fully validate the real-time PCR typing method, all samples were re-typed by PCR-SBT (sequence base typing). This analysis was performed as previously described (cf. S. Pozzi et al., "HLA-B locus sequence-based typing", Tissue Antigens 1999, vol. 53, pp. 275-8119) using FastStart™ polymerase (Roche Diagnostics GmbH) and BigDye™ Terminator Cycle Sequencing v2.0 (AbiPrism-PE Biosystems). In this approach PBX1 (sense) and BINT3 (antisense) were used as amplification primers for all locus B alleles with exception of HLA-B73. To detect this allele an additional amplification was performed with 18ClN3* as an antisense primer. The sequences of these primers are given in the reference cited. Results from PCR-SBT protocol were in all the cases in agreement with those obtained with the real-time PCR protocol (TABLE 3). All the 30 B27-negative samples by PCR-SBT where also negative with the new technique. Samples from some unusual B27-subtypes were not available.

**TABLE 1. Cell lines and previously known HLA B allele correspondency.**

| Cell line | HLA B ancestor 1 (one allele) | HLA B ancestor 2 (the other allele) |
|---|---|---|
| LH | 2701 | 08 |
| R34 | 2702 | 18 |
| CH | 2703 | 1801 |
| WE-WAK1 | 2704 | 1506 |
| HOM2 | 2705 | 2705 |
| LG2 | 2705 | 2705 |
| PAR | 2706 | 4801 |
| RQ | 2708 | 0801 |
| CI | 2709 | 3801 |

**TABLE 3. Concordance of results from genotyping and other typing methodologies used. Number of samples genotyped in each case is indicated.**

| PRC-SSP and PCR-SBT typing | FRET-real-time-PCR | Concordance |
|---|---|---|
| 28 (HLA-B2705/13) | 28 Group 8 (HLA-B2705/10/13/16/17) | 100% |
| 2 (HLA-B2702) | 2 Group 1 (HLA-B2702/01) | 100% |
| 30 (no-B27) | 30 Group 14 (no-B27) | 100% |

**TABLE 2. Primers and probes used in the reactions. All primers were from Sigma-Genosys (UK), except 127c and 362c from Tib-Mol Biol (Germany). In all cases, primer concentrations were reevaluated in the laboratory by spectrophotometer (Pharmacia LKB Ultrospec III). The numbers that identify each primer indicate positions 3' respect the cDNA. All hybriditzation probes were from Tib-Mol Biol.**

| Primer | Sequence (5'-> 3') | Reference | Orientation | Tm (° C) |
|---|---|---|---|---|
| 149C | TACGTGGACGACACGCT | SEQ ID NO: 1 | Sense | 61 |
| 273c | TGTGCCTTGGCCTTGC | SEQ ID NO: 22 | Antisense | 61 |
| 273C2 | GTGCCTTGGCCTTGC | SEQ ID NO: 2 | Antisense | 61 |
| 273c3 | AGTCTGTGTGTTGGTCTTGC | SEQ ID NO: 3 | Antisense | 60 |
| β-globin S | TACGGCTGTCATCACTTAGAC | RN 421104-24-7 GenBank AR181464 SEQ ID NO: 4 | Sense | 59 |
| β-globin AS | CTTCATCCACGTTCACCT | SEQ ID NO: 5 | Antisense | 62 |
| 127C | GCCCCGCTTCATCACC | SEQ ID NO: 6 | Sense | 64 |
| 362C | CACGTCGCAGCCATACATAT | SEQ ID NO: 7 | Antisense | 63 |
| 204 | GCCCGCGGCTCCTCT | SEQ ID NO: 8 | Antisense | 69 |
| 344c | TCCAGGGTCTCACACCC | SEQ ID NO: 9 | Sense | 62 |
| 462 | CGGCGGTCCAGGAGCT | RN 146415-96-5 SEQ ID NO: 10 | Antisense | 68 |
| 462d | CCGCGGTCCAGGAGCT | SEQ ID NO: 11 | Antisense | 60 |

**TABLE 2. Primers and probes used in the reactions (continuation).**

| FRET Probes | Sequence (5'-> 3') | Reference | Labelling | Tm (° C) |
|---|---|---|---|---|
| S1 sensor | AGAGGACCTGCCGGACCCTGC | SEQ ID NO: 12 | fluorescein | 69 |
| S1 anchor | GCTACTACAACCAGAGCGAGGCCGG | SEQ ID NO: 13 | LC-RED 640 | 72 |
| S2 sensor | GGAGCATTGGGACCGGGAG | SEQ ID NO: 14 | LC-RED 705 | 69 |
| S2 anchor | CGCCGTGGATAGAGCAGGAGGGG | SEQ ID NO: 15 | fluorescein | 72 |
| S3 sensor | GGTACCACCAGCACGCCT | SEQ ID NO: 16 | fluorescein | 68 |
| S3 anchor | CGACGGCAAGGATTACATCGCCCT | SEQ ID NO: 17 | LC-RED 640 | 72 |
| B27-Det_anchor | TGAGGTTCGACAGCGACGCC | SEQ ID NO: 18 | LC-red 640 | 72.3 |
| B27-Det_sensor | CGAGTCCGAGAGAGGAGCC | SEQ ID NO: 19 | fluorescein | 67,5 |
| CTW FL | CAAACAGACACCATGGTGCACCTGACTCCTGAGGA | SEQ ID NO: 20 | fluorescein | 75 |
| CTW LC | AAGTCTGCCGTTACTGCCCTGTGGGGCAA | SEQ ID NO: 21 | Lc-red 705 | 74 |

the different alleles of the allelic group, followed by a definitive definition based on an experimental assessment of which of the signals are actually positive for each melting temperature (black squares in FIG. 5), which are actually negative for each melting temperature (white squares in FIG. 5), and which are actually negative for lack of amplification (grey squares in FIG. 5).

## Claims

1. A method for determining alleles of the 27 allelic group of B locus of human leukocyte antigen (HLA-B27) from a nucleic acid sample, comprising the steps of:
(i) amplifying any nucleic acid that comprise a part of the region exon 2-intron 2-exon 3 of HLA-B locus by real-time polymerase chain reaction (PCR); and
(ii) detecting fluorescence signals either by fluorescently labelled nucleotide probes or by an intercalating agent, after the amplification carried out in step (i), and analysing melting temperatures of the amplified nucleic acid sequences;
followed, when detection in step (ii) has been done by fluorescently labelled nucleotide probes, by the step of
(iii) comparing more than one signal detected in step (ii) with an experimentally defined fluorescence pattern; said pattern having been established by an initial definition based on theoretical comparison of the sequences of probes of step (ii) with the sequences of the different alleles of the allelic group, followed by a definitive definition based on an experimental assessment of which of the signals are actually positive for each melting temperature (black squares in FIG. 5), which are actually negative for each melting temperature (white squares in FIG. 5) and which are actually negative for lack of amplification (grey squares in FIG. 5);
wherein in all cases steps (i) and (ii) are performed in the same tube and without any further manipulation of the sample.

2. The method according to claim 1, wherein the amplification of step (i) is carried out with two or more primers which are single strand nucleic acids, which have a length from 10 to 30 nucleotides, and which comprise sequences complementary to sequences of a region located along exon 2, intron 2 and 3 of HLA-B locus.

3. The method according to claim 2, wherein length of primers is from 14 to 24 nucleotides.

4. The method according to claim 3, wherein primer sequences are selected from the group consisting of SEQ ID NO: 1-3, SEQ ID NO: 6-11 and SEQ ID NO: 22.

5. The method according to any of the claims 1-4, wherein the intercalating agent of step (ii) is SYBR green I.

6. The method according to any of the claims 1-4, wherein the detection of steps (ii) and (iii) is carried out with one or more probes which are single strand nucleic acids, which have a length from 10 to 35 nucleotides, and which comprise sequences complementary to sequences of a region located along exon 2, intron 2 and exon 3 of HLA-B locus.

7. The method according to claim 6, wherein length of probes is from 15 to 27 nucleotides.

8. The method according to claim 7, wherein probe sequences are selected from the group consisting of SEQ ID NO: 12-21.

9. The method according to claim 7, wherein probe sequences are selected from the complementary sequences of the group consisting of SEQ ID NO: 12-21.

10. The method according to any of the claims 6-9, wherein probes are labelled with fluorochromes of type donor label-acceptor label.

11. The method according to any of the previous claims, wherein two primers for β-globin gene with SEQ ID NO: 4 and SEQ ID NO: 5 are added as a control in step (i).

12. A kit for determining alleles of the 27 allelic group of B locus of human leukocyte antigen (HLA-B27) from a nucleic acid sample by performing the method of claim 1, comprising:
(i) primers for amplifying nucleic acids that comprise a part of the region exon 2-intron 2-exon 3 of HLA-B locus by real-time polymerase chain reaction (PCR); and
(ii) either fluorescently labelled nucleotide probes or an intercalating agent, for detecting fluorescence signals after the amplification;
and, when fluorescently labelled nucleotide probes are included, further comprising
(iii) experimentally defined fluorescence patterns for comparing the detected signals, said patterns having been established by an initial definition based on theoretical comparison of the sequences of probes with the sequences of the different alleles of the allelic group, followed by a definitive definition based on an experimental assessment of which of the signals are actually positive for each melting temperature (black squares in FIG. 5), which are actually negative for each melting temperature (white squares in FIG. 5), or which are actually negative for lack of amplification (grey squares in FIG. 5).

13. The kit according to claim 12, wherein primers are single strand nucleic acids which have a length from 10 to 30 nucleotides and which comprise sequences complementary to sequences of a region located along exon 2, intron 2 and exon 3 of HLA-B locus.

14. The kit according to claim 13, wherein length of primers is from 14 to 24 nucleotides.

15. The kit according to claim 14, wherein primer sequences are selected from the group consisting of SEQ ID NO: 1-3, SEQ ID NO: 6-11 and SEQ ID NO: 22.

16. The kit according to any of the claims 12-15, wherein the intercalating agent is SYBR green I.

17. The kit according to any of the claims 12-15, wherein probes are single strand nucleic acids, which have a length from 10 to 35 nucleotides, and which comprise sequences complementary to sequences of a region located along exon 2, intron 2 and exon 3 of HLA-B locus.

18. The kit according to claim 17, wherein length of probes is from 15 to 27 nucleotides.

19. The kit according to claim 18, wherein probe sequences are selected from the group consisting of SEQ ID NO: 12-21.

20. The kit according to claim 18, wherein probe sequences are selected from the complementary sequences of the group consisting of SEQ ID NO: 12-21.

21. The kit according to any of the claims 17-20, wherein probes are labelled with fluorochromes of type donor label-acceptor label.

22. The kit according to any of the claims 12-21, wherein two primers for β-globin gene with SEQ ID NO: 4 and SEQ ID NO: 5 are added as a control.

23. A kit for determining alleles of the 27 allelic group of B locus of human leukocyte antigen (HLA-B27) from a nucleic acid sample by performing the method of claim 1, comprising:
(i) primers for amplifying nucleic acids that comprise a part of the region exon 2-intron 2-exon 3 of HLA-B locus by real-time polymerase chain reaction (PCR); these primers having sequences selected from the group consisting of SEQ ID NO: 1-3, SEQ ID NO: 6-11 and SEQ ID NO: 22;
(ii) fluorescently labelled nucleotide probes for detecting fluorescence signals after the amplification, these probes having sequences selected form the group consisting of SEQ ID NO: 12-21;
(iii) two primers for β-globin gene with SEQ ID NO: 4 and SEQ ID NO: 5 added as a control; and
(iii) experimentally defined fluorescence patterns for comparing the detected signals, said patterns having been established by an initial definition based on theoretical comparison of the sequences of probes with the sequences of
